# EUROPEAN PATENT SPECIFICATION

(11) **EP 1 591 823 B1**
(45) Date of publication and mention of the grant of the patent: **20.08.2008**
(21) Application number: 05252393.3
(22) Date of filing: 18.04.2005
(51) Int. Cl.: G02B 26/02

(54) **Optical shutter and light scanning apparatus employing the same**
Optischer Verschluss und Lichtabtastgerät, welches denselben benutzt
Obturateur optique et appareil de balayage optique l'utilisant

(30) Priority: 28.04.2004 KR 2004029673
(43) Date of publication of application: 02.11.2005
(73) Proprietor: SAMSUNG ELECTRONICS CO., LTD., Suwon-si, Gyeonggi-do (KR)
(72) Inventor: Kim, Sung-jin, Bundang-gu Seongnam-si Gyeonggi-do (KR); Shin, Seung-joo, Nowon-gu Seoul (KR); Moon, Il-kwon, Yeongtong-gu Suwon-si Gyeonggi-do (KR); Kwon, Kye-si, Seocho-gu Seoul (KR)
(74) Representative: Anderson, James Edward George

(56) References cited:
- EP-A- 1 069 450
- WO-A-03/005733
- WO-A-20/04027489
- US-A- 4 763 142
- US-A- 5 659 330
- US-A- 5 923 129
- US-A1- 2004 196 525

## Description

The present invention relates to a light scanning apparatus for an electrophotographic image forming apparatus, and more particularly, to an optical shutter which adjusts the amount of light transmitted using an electro-wetting phenomenon and an optical scanning apparatus employing the same.

In general, electrophotographic image forming apparatuses such as copiers, laser printers, and facsimiles produce desired images by forming a latent electrostatic image on a photosensitive medium such as a photoreceptor drum or photoreceptor belt, developing the latent electrostatic image with a developer of a predetermined color, and transferring the developed image to a print paper. The image forming apparatus includes a laser scanning unit (LSU) forming a latent electrostatic image corresponding to a desired image by scanning light onto a surface of the photosensitive medium.

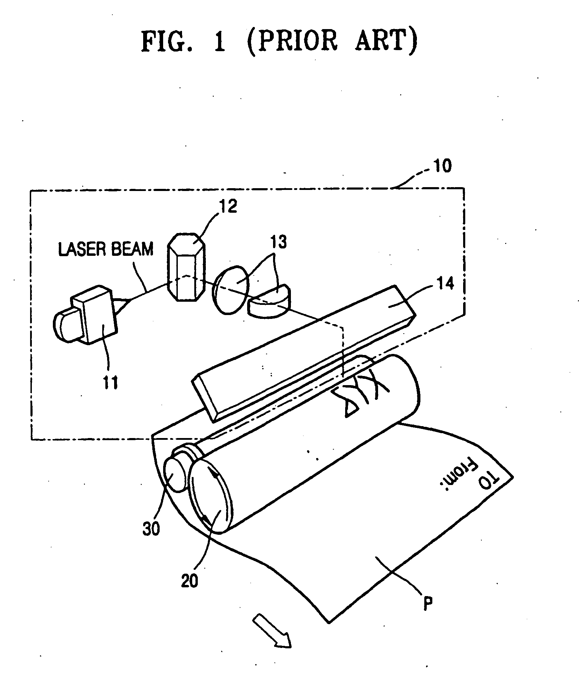

FIG. 1 shows the configuration of a light scanning apparatus for a conventional electrophotographic image forming apparatus.

Referring to FIG. 1, a conventional light scanning apparatus 10 includes a laser diode (LD) 11 as a light source for emitting a laser beam, a polygon mirror 12 scanning the laser beam emitted from the laser diode 11, a focusing lens 13 focusing the laser beam reflected by the polygon mirror 12, and a reflection mirror 14 reflecting the laser beam passing through the focusing lens 13 to form an image having a point shape on a surface of a photoreceptor drum 20 that is an image forming surface.

When the light scanning apparatus 10 scans a laser beam onto the surface of the photoreceptor drum 20 that is charged to a predetermined electric potential, electric charges in an area where the laser beam is scanned are lost. Thus, a latent electrostatic image having a different electric potential from that of the other area is formed in the area where the laser beam is scanned. Since toner supplied by a toner supply roller 30 is selectively attached to the latent electrostatic image by an electrostatic force, the latent electrostatic image is developed into a desired image. The image developed on the surface of the photoreceptor drum 20 is transferred to a print paper P and then fused on the print paper P by a fusing apparatus (not shown).

However, since the conventional light scanning apparatus 10 having the above structure includes an optical system having a complicated structure to scan a laser beam, the volume of the apparatus as well as manufacturing costs thereof increase. Also, since the conventional light scanning apparatus 10 has a structure to scan a laser beam while rotating the polygon mirror 12 using a motor (not shown), there is a limit in increasing a printing speed by reducing the time to scan a laser beam.

U.S. Patent No. 4,763,142 discloses a technology of forming a latent electrostatic image on a surface of a photoreceptor drum by adjusting transmissivity of light using a micro-shutter array using liquid crystal instead of the conventional light scanning apparatus. However, since a polarization panel is used for the shutter using liquid crystal, the transmissivity of light decreases so that power consumption of a light source increases.

Japanese Patent No. 7-120794 discloses an optical shutter using total reflection by bubbles generated in liquid that absorbs light. However, since the optical shutter requires a heating body to generate bubbles, manufacturing the optical shutter is difficult and power consumption increases.

WO 03/005733 A1 discloses an image projecting device comprising, in series: a light source, a diffractive element, a beam expander, a spatial light modulator employing liquid crystal cells, and magnification optics. U.S. Patent No. 5,659,330, EP 1069450 A2 and WO 2004/027489 all disclose an optical element, based on the electro-wetting phenomenon, for use in display devices. None of these documents, however, discloses electrophotographic applications.

According to an aspect of the present invention, there is provided an electrophotographic image forming apparatus comprising a light scanning apparatus, the light scanning apparatus comprising: at least one light source; an optical shutter array having a plurality of optical shutters arranged to adjust an amount of light emitted and transmitted from the light source; and a micro-lens array having a plurality of focusing lenses arranged to focus the light passing through each of the optical shutters, wherein the apparatus is characterized in that each of the optical shutters comprises: a cell having a transparent panel and a hydrophobic, transparent insulation panel arranged to face each other; a transparent electrode formed on an outer surface of the hydrophobic transparent insulation panel; and an opaque hydrophilic droplet contained in the cell for contact with an inner surface of the hydrophobic transparent insulation panel, wherein the amount of light transmitted by the optical shutter is adjustable by changing a contact angle of the hydrophilic droplet with respect to the hydrophobic transparent insulation panel, and wherein the contact angle is changeable by applying an electric field between the transparent electrode and the hydrophilic droplet.

The hydrophilic droplet is preferably formed by mixing a light shield material in water or electrolyte solution.

Preferably, a transparent hydrophobic liquid that is not mixed with the hydrophilic droplet is contained in the cell together with the hydrophilic droplet. The hydrophobic liquid may be formed of a non-conductive organic material. The organic material is preferably silicon oil.

According to another aspect of the present invention, there is provided an electrophotographic image forming apparatus comprising a light scanning apparatus, the light scanning apparatus comprising: at least one light source; an optical shutter array having a plurality of optical shutters arranged to adjust an amount of light emitted and transmitted from the light source; and a micro-lens array having a plurality of focusing lenses arranged to focus the light passing through each of the optical shutters, wherein the apparatus is characterized in that each of the optical shutters comprises: a cell having a transparent panel and a hydrophobic transparent insulation panel arranged to face each other; a transparent electrode formed on an outer surface of the hydrophobic transparent insulation panel; an opaque hydrophobic droplet contained in the cell for contact with an inner surface of the hydrophobic transparent insulation panel; and a transparent hydrophilic liquid contained in the cell and not mixed with the hydrophobic droplet, wherein the amount of light transmitted is adjustable by changing a contact angle of the hydrophobic droplet with respect to the hydrophobic transparent insulation panel, and wherein the contact angle is changeable by applying an electric field between the transparent electrode and the hydrophilic liquid.

The hydrophobic droplet is preferably formed by mixing a light shield material in a non-conductive organic material. The non-conductive organic material is preferably silicon oil. The hydrophilic liquid may be formed of water or electrolyte solution.

The optical shutters may be arranged in one row or zigzag in two rows.

The light source may be a single point light source. The point light source may be a laser diode or a light emitting diode. A collimator lens which converts light emitted from the point light source to a parallel beam may be arranged between the point light source and the optical shutter array.

The light source may alternatively be formed of a plurality of point light sources arranged with a predetermined interval. A plurality of collimator lenses which converts light emitted from the point light sources to a parallel beam may be arranged between the point light sources and the optical shutter array.

The light source may alternatively be formed of a single linear light source or a cold cathode fluorescent lamp.

The micro-lens array is preferably integrally formed with the optical shutter array.

The present invention thus provides optical shutter that can adjust the amount of light transmitted using an electro-wetting phenomenon, and a light scanning apparatus employing the same.

The above and other features and advantages of the present invention will become more apparent by describing in detail preferred embodiments thereof with reference to the attached drawings in which:
FIG. 1 is a perspective view illustrating the configuration of a conventional light scanning apparatus for an electrophotographic image forming apparatus;
FIG. 2 is a view illustrating the configuration of a light scanning apparatus according to a first embodiment of the present invention;
FIGS. 3A and 3B are enlarged views illustrating the configuration of an example of the optical shutter of FIG. 2, in which FIG. 3A shows a state in which light emitted from a light source passes through the optical shutter and FIG. 3B shows a state in which the light emitted from the light source is blocked by the optical shutter;
FIGS. 4A and 4B are enlarged views illustrating the configuration of a modified example of the optical shutter of FIG. 2, in which FIG. 4A shows a state in which light emitted from a light source is blocked by the optical shutter and FIG. 4B shows a state in which the light emitted from the light source passes through the optical shutter;
FIG. 5 is a view illustrating another arrangement of a plurality of optical shutters in the optical shutter array shown in FIG. 2;
FIG. 6 is a view illustrating the configuration of a light scanning apparatus according to a second embodiment of the present invention;
FIG. 7 is a view illustrating the configuration of a light scanning apparatus according to a third embodiment of the present invention; and
FIG. 8 is a view illustrating the configuration of a modified example of a micro-lens array of the light scanning apparatus according to the present invention.

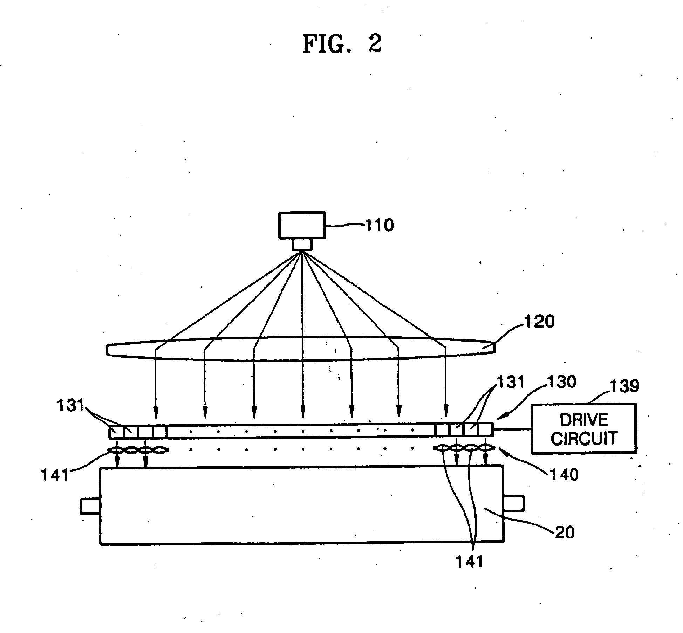

Referring to FIG. 2, a light scanning apparatus according to a first embodiment of the present invention, which forms a latent electrostatic image by scanning light on a surface of a photoreceptor medium, for example, the photoreceptor drum 20, of an electrophotographic image forming apparatus such as a laser printer, includes a point light source 110, an optical shutter array 130 having a plurality of optical shutters 131, and a micro-lens array 140 having a plurality of focusing lenses 141.

In the light scanning apparatus according to the present embodiment, a point light source is used as the light source 110 and a light emitting diode (LED) or a laser diode (LD) can be used as the point light source. In order to having a parallel light incident on the optical shutter array 130, a collimating lens 120 which converts the light emitted from the point light source 110 to a parallel light is arranged between the point light source 110 and the optical shutter array 130.

The optical shutters 131 of the optical shutter array 130 adjust the amount of the light emitted from the point light source 110 and transmitted. Each of the optical shutters 131 is independently and selectively driven by a drive circuit 139 and adjusts the amount of light transmitted through each of the optical shutters 131. The detailed structure and operation of each optical shutter will be described below.

In the optical shutter array 130, the optical shutters 131 may be arranged in a row. In this case, the number of the optical shutters 131 arranged in a row is the same as that of dots formed in one line of the surface of the photoreceptor drum 20 dependent on resolution of an image.

Since the optical shutters 131 can be simultaneously driven by the drive circuit 139, a latent electrostatic image corresponding to the dots of one line can be simultaneously formed on the surface of the photoreceptor drum 20. Thus, according to the optical scanning apparatus according to the present invention, since the time for scanning of a laser beam according to the conventional technology can be reduced so that a printing speed can be increased.

The focusing lenses 141 of the micro-lens array 140 focus the light passing through the optical shutters 131. The focusing lenses 141 are arranged to corresponding to the arrangement of the optical shutters 131.

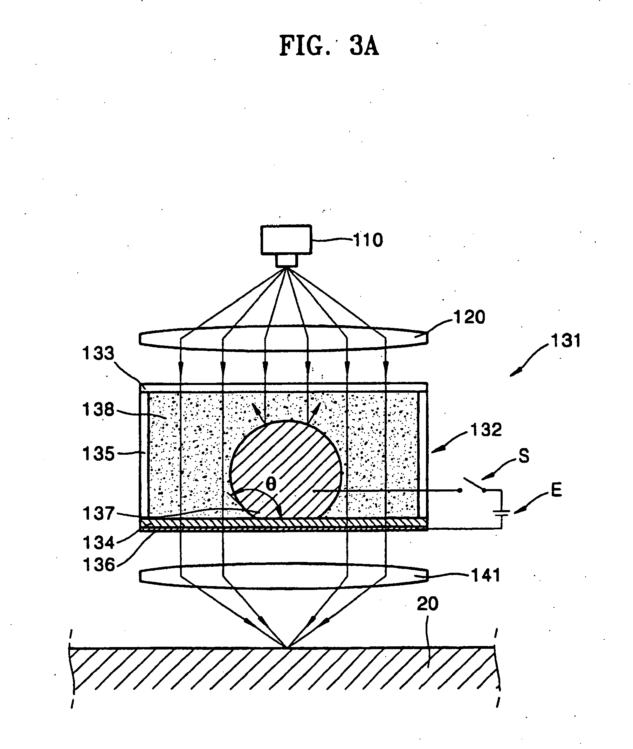

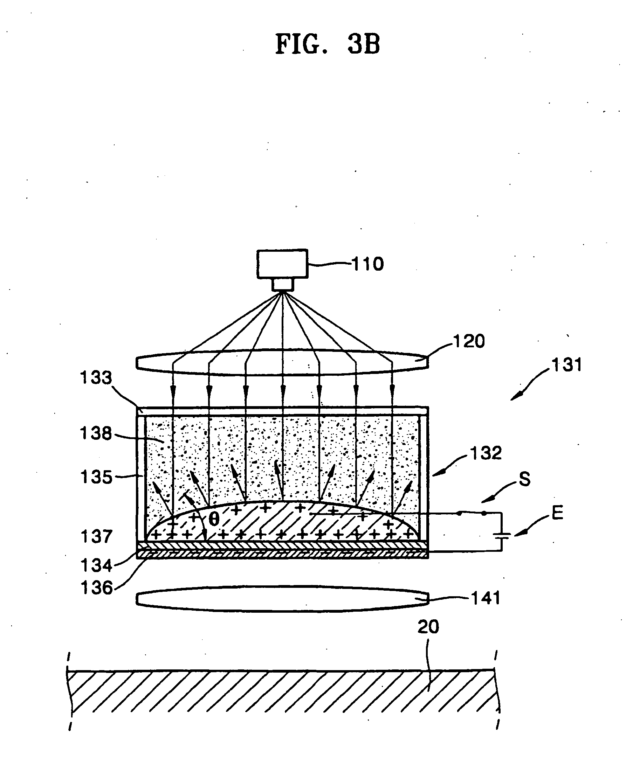

FIGS. 3A and 3B are enlarged views illustrating the configuration of an example of the optical shutter of FIG. 2, in which FIG. 3A shows a state in which light emitted from a light source passes through the optical shutter and FIG. 3B shows a state in which the light emitted from the light source is blocked by the optical shutter.

Referring to FIG. 3A, the optical shutters 131 having the same structure are provided in the optical shutter array 130. Each of the optical shutters 131 includes a cell 132 having an inner space which is sealed by being encompassed by two panels 133 and 134 arranged to face each other and side walls 135. One of the two panels 133 and 134, for example, the panel 133 in the present embodiment, may be formed of a transparent panel so that light can pass therethrough. A glass panel, for example, can be used as the transparent panel 133. The other panel, for example, the panel 134 in the present embodiment, is formed of a hydrophobic transparent insulation panel having a surface having hydrophobic and insulation properties and simultaneously a light transmission property. The hydrophobic transparent insulation panel 134 may be entirely formed of a hydrophobic material that is transparent and insulating. Alternatively, the hydrophobic transparent insulation panel 134 may be formed by coating a surface of the glass panel with a hydrophobic material to a predetermined thickness. The side walls 135 maintain interval between the transparent panel 133 and the hydrophobic transparent insulation panel 134 and seals the inner space of the cell 132. It is not necessary for the side walls 135 to be transparent.

An electrode 136 is formed on an outer surface of the hydrophobic transparent insulation panel 134. The electrode 136 may be formed of a transparent electrode material, for example, indium tin oxide (ITO), so as to be conductive and capable of transmitting light.

A droplet 137 that is hydrophilic is contained in the inner space of the cell 132. The droplet 137 contacts an inner surface of the hydrophobic transparent insulation panel 134 at a predetermined contact angel θ. The contact angle θ is a relatively large value due to different surface properties between the hydrophobic transparent insulation panel 134 and the hydrophilic droplet 137. The hydrophilic droplet 137 is formed of a material that is conductive and opaque to block light. The hydrophilic droplet 137 having the above properties can be made by mixing a light shield material that absorbs or reflects light, for example, black pigment, in water or electrolyte solution.

An electric source E to apply an electric field and a switch S to cut the electric source E are connected between the transparent electrode 136 and the hydrophilic droplet 137.

A portion of the inner space of the cell 132 which is not occupied by the hydrophilic droplet 137 can be filled with air or a transparent hydrophobic liquid 138 that is not mixed with the hydrophilic droplet 137. The hydrophobic liquid 138 can be formed of a non-conductive organic material, for example, silicon oil.

The operation of the optical shutters 131 having the above structure will be described below.

As shown in FIG. 3A, when an electric field is not applied between the transparent electrode 136 and the hydrophilic droplet 137, the hydrophilic droplet 137 is in contact with the surface of the hydrophobic transparent insulation panel 134 at a relatively large contact angle θ and has an almost ball shape due to a surface tension thereof. Thus, part of light emitted from the point light source 110 and passing through the collimating lens 120 is absorbed or reflected by the opaque hydrophilic droplet 137 so as not to pass through the optical shutters 131. However, most light can arrive at the surface of the photoreceptor drum 20 by passing through the transparent hydrophobic liquid 138. That is, when an electric field is not applied between the transparent electrode 136 and the hydrophilic droplet 137, the transmissivity of light passing through the optical shutters 131 has a relatively high value.

The light passing through the optical shutters 131 with a high transmissivity arrives at the surface of the photoreceptor drum 20 to form a latent electrostatic image. Since the light passing through the optical shutters 131 is focused by the focusing lens 141, the diameter of a light spot formed on the surface of the photoreceptor drum 20 decreases and accordingly resolution of an image can be improved.

Referring to FIG. 3B, when an electric field is applied between the transparent electrode 136 and the hydrophilic droplet 137, the contact angle θ of the hydrophilic droplet 137 with respect to the hydrophobic transparent insulation panel 134 decreases due to an electro-wetting phenomenon. Accordingly, the hydrophilic droplet 137 contacts a large area of the surface of the hydrophobic transparent insulation panel 134. In detail, when an electric field is applied between the transparent electrode 136 and the hydrophilic droplet 137, negative charges are accumulated in the transparent electrode 136 and positive charges are accumulated in the hydrophilic droplet 137 with the hydrophobic transparent insulation panel 134 interpose therebetween. Thus, the hydrophilic droplet 137 spreads widely by an electrostatic force between the negative charges and the positive charges.

When the hydrophilic droplet 137 spreads widely, the entire or most light emitted from the point light source 110 and passing through the collimator lens 120 is absorbed or reflected by the opaque hydrophilic droplet 137 so as not to pass through the optical shutters 131.

In the optical shutters 131 according to the present invention, the contact angle θ of the hydrophilic droplet 137 with respect to the hydrophobic transparent insulation panel 134 changes according to the application of an electric field between the transparent electrode 136 and the hydrophilic droplet 137. As a result, the amount of light transmitted is adjusted.

In the light scanning apparatus according to the present invention, since the optical shutter array 130 having the optical shutters 131 is provided, the structure of the light scanning apparatus is simplified compared to the conventional light scanning apparatus having a polygon mirror, a motor that rotates the polygon mirror, and a plurality of lenses and mirrors for laser scanning. Also, since the optical shutters 131 according to the present invention do not use a polarization panel unlike the conventional optical shutter using liquid crystal, the transmissivity of light increases so that power consumption of the point light source 110 is lowered.

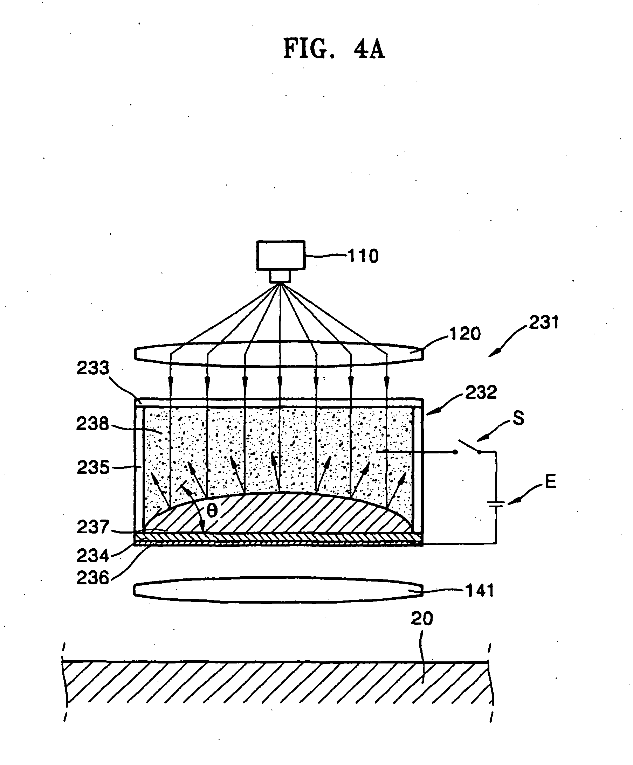

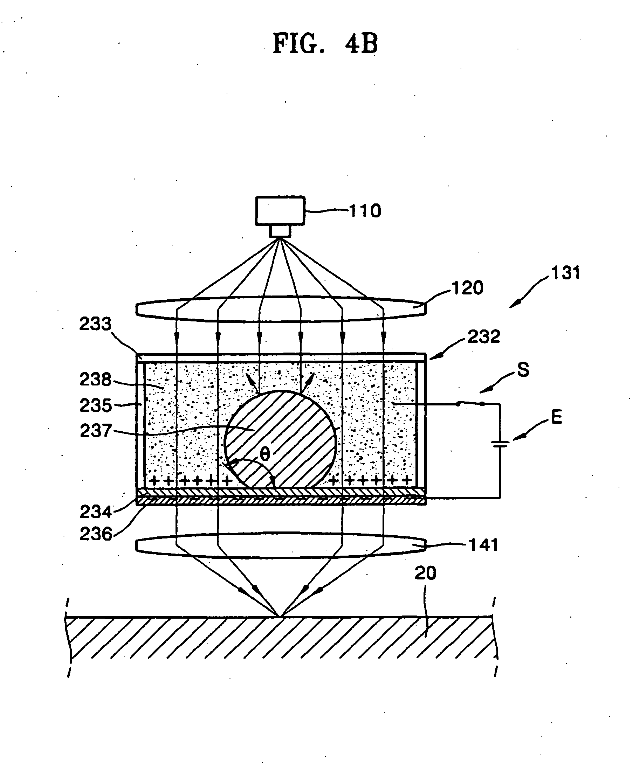

FIGS. 4A and 4B are enlarged views illustrating the configuration of a modified example of the optical shutter of FIG. 2, in which FIG. 4A shows a state
in which light emitted from a light source is blocked by the optical shutter and FIG. 4B shows a state in which the light emitted from the light source passes through the optical shutter.

Referring to FIG. 4A, an optical shutter 231 according to a modified example of the present invention includes a cell 232 having an inner space sealed by being encompassed by a transparent panel 233 and a hydrophobic transparent insulation panel 234, which are arranged to face each other, and side walls 235. A transparent electrode 236 is formed on an outer surface of the hydrophobic transparent insulation panel 234. Since the structures of the transparent panel 233, the hydrophobic transparent insulation panel 234, the side walls 235, and the transparent electrode 236 are the same as those of the optical shutters 131 shown in FIG. 3A, detailed descriptions thereof will be omitted herein.

A droplet 237 that is hydrophobic and liquid 238 that is hydrophilic are contained in the inner space of the cell 232. The hydrophobic droplet 237 contacts an inner surface of the hydrophobic transparent insulation panel 234 at a predetermined contact angle θ. Here, since the contact angle θ is a relatively small value because the hydrophobic transparent insulation panel 234 and the hydrophobic droplet 237 have the same surface property, the hydrophobic droplet 237 contacts a large area of the surface of the hydrophobic transparent insulation panel 234. The hydrophobic droplet 237 is formed of a material that is opaque and non-conductive so as not to transmit light. The hydrophobic droplet 237 having the above property can be formed by mixing a light shield material, for example, black pigment, in a non-conductive organic material, for example, silicon oil.

The hydrophilic liquid 238 is formed of a material that is conductive and transparent and is not mixed with the hydrophobic droplet 237. The hydrophilic liquid 238 having the above properties can be made of water or electrolyte solution. The electric source E to apply an electric field and the switch S to cut the electric source E are connected between the transparent electrode 236 and the hydrophilic liquid 238.

The operation of the optical shutter 231 having the above structure will be described below.

As shown in FIG. 4A, when an electric field is not applied between the transparent electrode 236 and the hydrophilic liquid 238, the hydrophobic droplet 237 having the same surface property as that of the hydrophobic transparent insulation panel 234 is in contact with the surface of the hydrophobic transparent insulation panel 234 in a widely spread state. Accordingly, the entire or most of light emitted from the point light source 110 and passing through the collimating lens 120 is absorbed or reflected by the opaque hydrophobic droplet 237 so as not to pass through the optical shutter 231.

Referring to FIG. 4B, when an electric field is applied to the transparent electrode 236 and the hydrophilic liquid 238 by connecting the electric source E thereto, a contact area between the hydrophilic liquid 238 and the hydrophobic transparent insulation panel 234 increases by an electro-wetting phenomenon. Accordingly, a contact area between the hydrophobic droplet 237 and the hydrophobic transparent insulation panel 234 decreases so that the contact angle θ of the hydrophobic droplet 237 with respect to the hydrophobic transparent insulation panel 234 increases. In detail, when an electric field is applied between the transparent electrode 236 and the hydrophobic liquid 238, negative charges are accumulated in the transparent electrode 236 and positive charges are accumulated in the hydrophilic liquid 238 with the hydrophobic transparent insulation panel 234 interpose therebetween. As the hydrophilic liquid 238 pushes the hydrophobic droplet 237 and moves toward the hydrophobic transparent insulation panel 234 by an electrostatic force between the negative charges and the positive charges. Thus, while the contact area between the hydrophilic liquid 238 and the hydrophobic transparent insulation panel 234 increases, the contact area between the hydrophobic droplet 237 and the hydrophobic transparent insulation panel 234 decreases.

Therefore, most of the light emitted from the point light source 110 and passing through the collimating lens 120 passes through the transparent hydrophilic liquid 238 to arrive at the surface of the photoreceptor drum 20. That is, when an electric field is applied to the transparent electrode 236 and the hydrophilic liquid 238, the transmissivity of light passing through the optical shutter 231 has a relatively high value. Also, the light passing through the optical shutter 231 is focused by the focusing lens 141 to form a light spot having a relatively small diameter on the surface of the photoreceptor drum 20.

In the above-described modified example of the optical shutter 231, the contact angle of the hydrophobic droplet 237 with respect to the hydrophobic transparent insulation panel 234 changes dependent on the application of the electric field between the transparent electrode 236 and the hydrophilic liquid 238. Accordingly, the amount of light transmitted is adjusted.

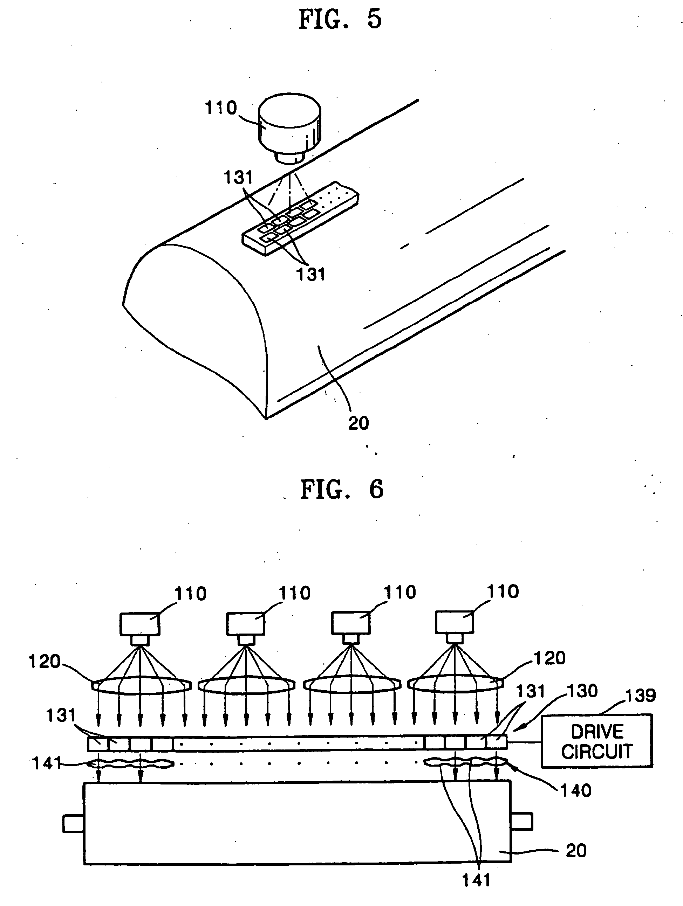

FIG. 5 is a view illustrating another arrangement of a plurality of optical shutters in the optical shutter array shown in FIG. 2. Referring to FIG. 5, in the optical shutter array 130, the optical shutters 131 may be arranged zigzag in two rows. The number of the optical shutters arranged in two rows is the same as that of the dots formed in one line on the surface of the photoreceptor drum 20. Such arrangement may be used for a case in which all the optical shutters 131 cannot be arranged in one row because the size of each of the optical shutters 131 is large.

FIG. 6 is a view illustrating the configuration of a light scanning apparatus according to a second embodiment of the present invention. Referring to FIG. 6, a light scanning apparatus according to a second embodiment of the present invention includes a plurality of point light sources 110 arranged with a predetermined interval. Laser diodes or light emitting diodes can be used as the point light sources 110. A plurality of collimator lenses 120 which convert light emitted from the point light sources 110 to a parallel beam are arranged between the point light sources 110 and the optical shutter array 130.

When the point light sources 110 are used, the interval between the point light sources 110 and the optical shutter array 130 can be further reduced compared to a case in which only one point light source is used. Thus, a more compact light scanning apparatus can be realized. Here, other constituent elements of the light scanning apparatus according to the present embodiment are the same as those of the light scanning apparatus according to the first embodiment of the present invention.

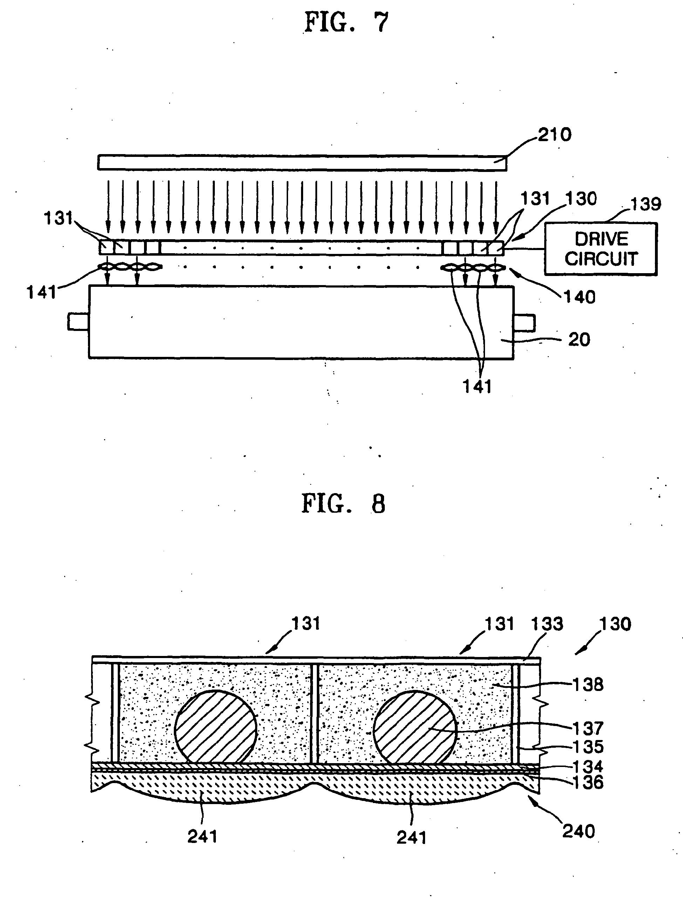

FIG. 7 is a view illustrating the configuration of a light scanning apparatus according to a third embodiment of the present invention. Referring to FIG. 7, a light scanning apparatus according to a third embodiment of the present invention includes one linear light source 210. Cold cathode fluorescent lamp (CCFL) may be typically used as the linear light source 210.

When the linear light source 210 is used, a parallel beam can be incident on the optical shutter array 130 if the collimator lens 120 shown in FIGS. 2 and 6 do not exist. Thus, a light scanning apparatus can be made compacter than in a case where the point light source 110 is used. Here, other constituent elements of the light scanning apparatus according to the present embodiment are the same as those of the light scanning apparatus according to the first and second embodiments of the present invention.

FIG. 8 is a view illustrating the configuration of a modified example of a micro-lens array of the light scanning apparatus according to the present invention. Referring to FIG. 8, a micro-lens array 240 having a plurality of focusing lenses 241 for focusing light which passes through each of the optical shutters 131 can be integrally formed with the optical shutter array 130.

When the micro-lens array 240 is integrally formed with the optical shutter array 130, manufacturing and handling of the micro-lens array 240 is made easy so that the size and manufacturing costs of the optical scanning apparatus can be lowered.

As described above, since the light scanning apparatus according to the present invention includes the optical shutter which adjusts the amount of light transmitted using the electro-wetting phenomenon, the structure thereof becomes simple. Thus, a compact light scanning apparatus can be realized with less manufacturing costs.

Also, since the conventional scanning time of a laser beam can be reduced, printing speed can be increased. Furthermore, since the optical shutter according to the present invention does not need a polarization panel unlike the convention optical shutter using liquid crystal, the transmissivity of light is improved so that power consumption of the light source is lowered.

While this invention has been particularly shown and described with reference to preferred embodiments thereof, it will be understood by those skilled in the art that various changes in form and details may be made therein without departing from the scope of the invention as defined by the appended claims.

## Claims

1. An electrophotographic image forming apparatus comprising a light scanning apparatus, the light scanning apparatus comprising:
at least one light source (110);
an optical shutter array (130) having a plurality of optical shutters (131) arranged to adjust an amount of light emitted and transmitted from the light source (110); and
a micro-lens array (140) having a plurality of focusing lenses (141) arranged to focus the light passing through each of the optical shutters (131),
wherein the apparatus is **characterized in that** each of the optical shutters (131) comprises:
a cell (132) having a transparent panel (133) and a hydrophobic transparent insulation panel (134) arranged to face each other;
a transparent electrode (136) formed on an outer surface of the hydrophobic transparent insulation panel (134); and
an opaque hydrophilic droplet (137) contained in the cell (132) for contact with an inner surface of the hydrophobic transparent insulation panel (134),
wherein the amount of light transmitted by the optical shutter (131) is adjustable by changing a contact angle of the hydrophilic droplet (137) with respect to the hydrophobic transparent insulation panel (134), and wherein the contact angle is changeable by applying an electric field between the transparent electrode (136) and the hydrophilic droplet (137).

2. The apparatus as claimed in claim 1, wherein the hydrophilic droplet (137) comprises a light shield material mixed with water or electrolyte solution.

3. The apparatus as claimed in claim 1 or 2, wherein a transparent hydrophobic liquid (138) that is not mixed with the hydrophilic droplet (137) is contained in the cell (132) together with the hydrophilic droplet (137).

4. The apparatus as claimed in claim 3, wherein the hydrophobic liquid (138) is formed of a non-conductive organic material.

5. The apparatus as claimed in claim 4, wherein the organic material is silicon oil.

6. An electrophotographic image forming apparatus comprising a light scanning apparatus, the light scanning apparatus comprising:
at least one light source (110);
an optical shutter array having a plurality of optical shutters (231) arranged to adjust an amount of light emitted and transmitted from the light source (110); and
a micro-lens array having a plurality of focusing lenses (141) arranged to focus the light passing through each of the optical shutters (231),
wherein the apparatus is **characterized in that** each of the optical shutters (231) comprises:
a cell (232) having a transparent panel (233) and a hydrophobic transparent insulation panel (234) arranged to face each other;
a transparent electrode (236) formed on an outer surface of the hydrophobic transparent insulation panel (234);
an opaque hydrophobic droplet (237) contained in the cell (232) for contact with an inner surface of the hydrophobic transparent insulation panel (234); and
a transparent hydrophilic liquid (238) contained in the cell (232) and not mixed with the hydrophobic droplet (237),
wherein the amount of light transmitted is adjustable by changing a contact angle of the hydrophobic droplet (237) with respect to the hydrophobic transparent insulation panel (234), and wherein the contact angle is changeable by applying an electric field between the transparent electrode (236) and the hydrophilic liquid (238).

7. The apparatus as claimed in claim 6, wherein the hydrophobic droplet (237) comprises a light shield material mixed with a non-conductive organic material.

8. The apparatus as claimed in claim 7, wherein the non-conductive organic material is silicon oil.

9. The apparatus as claimed in any of claims 6 to 8, wherein the hydrophilic liquid (238) is formed of water or electrolyte solution.

10. The apparatus as claimed in any preceding claim, wherein the optical shutters (231) are arranged in one row.

11. The apparatus as claimed in any of claims 1 to 9, wherein the optical shutters (231) are arranged zigzag in two rows.

12. The apparatus as claimed in any of the preceding claims, wherein the light source (110) is a single point light source (110).

13. The apparatus as claimed in claim 12, wherein the point light source (110) is a laser diode or a light-emitting diode.

14. The apparatus as claimed in claim 12 or 13, wherein a collimator lens (120) arranged to convert light emitted from the point light source (110) to a parallel beam is arranged between the point light source (110) and the optical shutter array (130).

15. The apparatus as claimed in any of claims 1 to 11, wherein the light source (110) comprises a plurality of point light sources (110) arranged with a predetermined interval.

16. The apparatus as claimed in claim 15, wherein the point light sources (110) are laser diodes or light emitting diodes.

17. The apparatus as claimed in claim 15 or 16, wherein a plurality of collimator lenses (120) arranged to convert light emitted from the point light sources (110) to a parallel beam are arranged between the point light sources (110) and the optical shutter array (130).

18. The apparatus as claimed in any of claims 1 to 9, wherein the light source is formed of a single linear light source (210).

19. The apparatus as claimed in claim 18, wherein the linear light source (210) is a cold cathode fluorescent lamp.

20. The apparatus as claimed in any preceding claim, wherein the micro-lens array (140) is integrally formed with the optical shutter array (130).

## Patentansprüche

1. Elektrophotographisches Bildgebungsgerät umfassend ein Lichtabtastgerät, wobei das Lichtabtastgerät umfasst:
mindestens eine Lichtquelle (110),
eine optische Verschlussanordnung (130) mit einer Mehrzahl von optischen Verschlüssen (131), die so ausgebildet sind, dass sie eine von der Lichtquelle (110) emittierte und transmittierte Lichtmenge einstellen, und
eine Mikrolinsenanordnung (140) mit einer Mehrzahl von Fokussierlinsen (141), die so ausgebildet sind, dass sie das jeden der optischen Verschlüsse (131) passierende Licht fokussieren,
wobei das Gerät **dadurch gekennzeichnet ist, dass** jeder der optischen Verschlüsse (131) umfasst:
eine Zelle (132) mit einer transparenten Platte (133) und einer hydrophoben transparenten Isolierplatte (134), die so angeordnet sind, dass sie einander zugewandt sind,
eine transparente Elektrode (136), die auf einer Außenseite der hydrophoben transparenten Isolierplatte (134) ausgebildet ist, und
ein lichtundurchlässiges hydrophiles Tröpfchen (137), das in der Zelle (132) enthalten ist, zum Kontakt mit einer Innenseite der hydrophoben transparenten lsolierplatte (134),
wobei die vom optischen Verschluss (131) transmittierte Lichtmenge durch Verändern eines Kontaktwinkels des hydrophilen Tröpfchens (137) zur hydrophoben transparenten Isolierplatte (134) einstellbar ist und wobei der Kontaktwinkel durch Anlegen eines elektrischen Feldes zwischen der transparenten Elektrode (136) und dem hydrophilen Tröpfchen (137) veränderbar ist.

2. Gerät nach Anspruch 1, wobei das hydrophile Tröpfchen (137) ein Lichtabschirmungsmaterial vermischt mit Wasser oder Elektrolytlösung umfasst.

3. Gerät nach Anspruch 1 oder 2, wobei eine transparente hydrophobe Flüssigkeit (138), die nicht mit dem hydrophilen Tröpfchen (137) vermischt ist, in der Zelle (132) zusammen mit dem hydrophilen Tröpfchen (137) enthalten ist.

4. Gerät nach Anspruch 3, wobei die hydrophobe Flüssigkeit (138) aus einem nicht leitenden organischen Material gebildet ist.

5. Gerät nach Anspruch 4, wobei das organische Material Siliconöl ist.

6. Elektrophotographisches Bildgebungsgerät umfassend ein Lichtabtastgerät, wobei das Lichtabtastgerät umfasst:
mindestens eine Lichtquelle (110),
eine optische Verschlussanordnung mit einer Mehrzahl von optischen Verschlüssen (231), die so ausgebildet sind, dass sie eine von der Lichtquelle (110) emittierte und transmittierte Lichtmenge einstellen, und
eine Mikrolinsenanordnung mit einer Mehrzahl von Fokussierlinsen (141), die so ausgebildet sind, dass sie das jeden der optischen Verschlüsse (231) passierende Licht fokussieren,
wobei das Gerät **dadurch gekennzeichnet ist, dass** jeder der optischen Verschlüsse (231) umfasst:
eine Zelle (232) mit einer transparenten Platte (233) und einer hydrophoben transparenten Isolierplatte (234), die so angeordnet sind, dass sie einander zugewandt sind,
eine transparente Elektrode (236), die auf einer Außenseite der hydrophoben transparenten Isolierplatte (234) ausgebildet ist,
ein lichtundurchlässiges hydrophobes Tröpfchen (237), das in der Zelle (232) enthalten ist, zum Kontakt mit einer. Innenseite der hydrophoben transparenten Isolierplatte (234) und
eine transparente hydrophile Flüssigkeit (238), die in der Zelle (232) enthalten und nicht mit dem hydrophoben Tröpfchen (237) vermischt ist,
wobei die transmittierte Lichtmenge durch Verändern eines Kontaktwinkels des hydrophoben Tröpfchens (237) zur hydrophoben transparenten Isolierplatte (234) einstellbar ist und wobei der Kontaktwinkel durch Anlegen eines elektrischen Feldes zwischen der transparenten Elektrode (236) und der hydrophilen Flüssigkeit (238) veränderbar ist.

7. Gerät nach Anspruch 6, wobei das hydrophobe Tröpfchen (237) ein Lichtabschirmungsmaterial vermischt mit einem nicht leitenden organischen Material umfasst.

8. Gerät nach Anspruch 7, wobei das nicht leitende organische Material Siliconöl ist.

9. Gerät nach einem der Ansprüche 6 bis 8, wobei die hydrophile Flüssigkeit (238) aus Wasser oder Elektrolytlösung gebildet ist.

10. Gerät nach einem der vorhergehenden Ansprüche, wobei die optischen Verschlüsse (231) in einer Reihe angeordnet sind.

11. Gerät nach einem der Ansprüche 1 bis 9, wobei die optischen Verschlüsse (231) in zwei Reihen im Zickzack angeordnet sind.

12. Gerät nach einem der vorhergehenden Ansprüche, wobei die Lichtquelle (110) eine einzelne Punktlichtquelle (110) ist.

13. Gerät nach Anspruch 12, wobei die Punktlichtquelle (110) eine Laserdiode oder eine Lichtemissionsdiode ist.

14. Gerät nach Anspruch 12 oder 13, wobei eine Sammellinse (120), die so ausgebildet ist, dass sie von der Punktlichtquelle (110) emittiertes Licht in einen parallelen Strahl konvertiert, zwischen der Punktlichtquelle (110) und der optischen Verschlussanordnung (130) angeordnet ist.

15. Gerät nach einem der Ansprüche 1 bis 11, wobei die Lichtquelle (110) eine Mehrzahl von Punktlichtquellen (110) umfasst, die in einem vorgegebenen Intervall angeordnet sind.

16. Gerät nach Anspruch 15, wobei die Punktlichtquellen (110) Laserdioden oder Lichtemissionsdioden sind.

17. Gerät nach Anspruch 15 oder 16, wobei eine Mehrzahl von Sammellinsen (120), die so ausgebildet sind, dass sie von den Punktlichtquellen (110) emittiertes Licht in einen parallelen Strahl konvertieren, zwischen den Punktlichtquellen (110) und der optischen Verschlussanordnung (130) angeordnet sind.

18. Gerät nach einem der Ansprüche 1 bis 9, wobei die Lichtquelle aus einer einzelnen Linearlichtquelle (210) gebildet ist.

19. Gerät nach Anspruch 18, wobei die Linearlichtquelle (210) eine Kaltkathoden-Fluoreszenzlampe ist.

20. Gerät nach einem der vorhergehenden Ansprüche, wobei die Mikrolinsenanordnung (140) integral mit der optischen Verschlussanordnung (130) ausgebildet ist.

## Revendications

1. Appareil de formation d'image électro-photographique comprenant un appareil de balayage optique, l'appareil de balayage optique comprenant :
au moins une source de lumière (110) ;
une matrice d'obturateurs optiques (130) ayant une pluralité d'obturateurs optiques (131) agencés pour ajuster une quantité de lumière émise et transmise depuis la source de lumière (110) ; et
une matrice de micro-lentilles (140) ayant une pluralité de lentilles de focalisation (141) agencées pour focaliser la lumière passant à travers chacun des obturateurs optiques (131),
dans lequel l'appareil est **caractérisé en ce que** chacun des obturateurs optiques (131) comprend :
une cellule (132) ayant un panneau transparent (133) et un panneau d'isolation transparent hydrophobe (134) agencé pour se faire face l'un l'autre ;
une électrode transparente (136) formée sur une surface extérieure du panneau d'isolation transparent hydrophobe (134) ; et
une gouttelette opaque hydrophile (137) contenue dans la cellule (132) pour un contact avec une surface intérieure du panneau d'isolation transparent hydrophobe (134),
dans lequel la quantité de lumière transmise par l'obturateur optique (131) est ajustable en changeant un angle de contact de la gouttelette hydrophile (137) par rapport au panneau d'isolation transparent hydrophobe (134), et dans lequel l'angle de contact est changeable en appliquant un champ électrique entre l'électrode transparente (136) et la gouttelette hydrophile (137).

2. Appareil tel que revendiqué dans la revendication 1, dans lequel la gouttelette hydrophile (137) comprend un matériau écran optique mélangé avec de l'eau ou une solution électrolyte.

3. Appareil tel que revendiqué dans la revendication 1 ou 2, dans lequel un liquide transparent hydrophobe (138) qui n'est pas mélangé à la gouttelette hydrophile (137) est contenu dans la cellule (132) avec la gouttelette hydrophile (137).

4. Appareil tel que revendiqué dans la revendication 3, dans lequel le liquide hydrophobe (138) est formé d'un matériau non-conducteur organique.

5. Appareil tel que revendiqué dans la revendication 4, dans lequel le matériau organique est de l'huile de silicone.

6. Appareil de formation d'image électro-photographique comprenant un appareil de balayage optique, l'appareil de balayage optique comprenant :
au moins une source de lumière (110) ;
une matrice d'obturateurs optiques ayant une pluralité d'obturateurs optiques (231) agencés pour ajuster une quantité de lumière émise et transmise depuis la source de lumière (110) ; et
une matrice de micro-lentilles ayant une pluralité de lentilles de focalisation (141) agencées pour focaliser la lumière passant à travers chacun des obturateurs optiques (231),
dans lequel l'appareil est **caractérisé en ce que** chacun des obturateurs optiques (231) comprend :
une cellule (232) ayant un panneau transparent (233) et un panneau d'isolation transparent hydrophobe (234) agencé pour se faire face l'un l'autre ;
une électrode transparente (236) formée sur une surface extérieure du panneau d'isolation transparent hydrophobe (234) ; et
une gouttelette opaque hydrophobe (237) contenue dans la cellule (232) pour un contact avec une surface intérieure du panneau d'isolation transparent hydrophobe (234),
un liquide transparent hydrophile (238) contenu dans la cellule (232) et non mélangé avec la gouttelette hydrophobe (237),
dans lequel la quantité de lumière transmise est ajustable en changeant un angle de contact de la gouttelette hydrophobe (237) par rapport au panneau d'isolation transparent hydrophobe (234), et dans lequel l'angle de contact est changeable en appliquant un champ électrique entre l'électrode transparente (236) et le liquide hydrophile (238).

7. Appareil tel que revendiqué dans la revendication 6, dans lequel la gouttelette hydrophobe (237) comprend un matériau écran optique mélangé avec un matériau non conducteur organique.

8. Appareil tel que revendiqué dans la revendication 7, dans lequel le matériau organique est de l'huile de silicone.

9. Appareil tel que revendiqué dans l'une quelconque des revendications 6 à 8, dans lequel le liquide hydrophile (238) est formé d'eau ou d'une solution électrolyte.

10. Appareil tel que revendiqué dans l'une quelconque des revendications précédentes, dans lequel les obturateurs optiques (231) sont agencés en une colonne.

11. Appareil tel que revendiqué dans l'une quelconque des revendications 1 à 9, dans lequel les obturateurs optiques (231) sont agencés en zigzag en deux colonnes.

12. Appareil tel que revendiqué dans l'une quelconque des revendications précédentes, dans lequel la source de lumière (110) est une unique source de lumière ponctuelle (110).

13. Appareil tel que revendiqué dans la revendication 12, dans lequel la source de lumière ponctuelle (110) est une diode laser ou une diode électroluminescente.

14. Appareil tel que revendiqué dans la revendication 12 ou 13, dans lequel une lentille de collimateur (120) configurée pour convertire la lumière émise depuis la source de lumière ponctuelle (110) en un faisceau parallèle est agencée entre la source de lumière ponctuelle (110) et la matrice d'obturateurs optiques (130).

15. Appareil tel que revendiqué dans l'une quelconque des revendications 1 à 11, dans lequel la source de lumière comprend une pluralité de sources de lumière ponctuelles (110) agencées dans un intervalle prédéterminé.

16. Appareil tel que revendiqué dans la revendication 15, dans lequel les sources de lumière ponctuelle (110) sont des diodes laser ou des diodes électroluminescentes.

17. Appareil tel que revendiqué dans la revendication 15 ou 16, dans lequel une pluralité de lentilles de collimateur (120) configurées pour convertir la lumière émise depuis les sources de lumière ponctuelles (110) en un faisceau parallèle sont agencées entre les sources de lumière ponctuelles (110) et la matrice d'obturateurs optiques (130).

18. Appareil tel que revendiqué dans l'une quelconque des revendications 1 à 9, dans lequel la source de lumière est formée par une unique source de lumière linéaire (210).

19. Appareil tel que revendiqué dans la revendication 18, dans lequel la source de lumière linéaire (210) est une lampe fluorescente à cathode froide.

20. Appareil tel que revendiqué dans l'une quelconque des revendications précédentes, dans lequel la matrice de micro-lentilles (140) est formée intégralement avec la matrice d'obturateurs optiques (130).
